# EUROPEAN PATENT SPECIFICATION

(11) **EP 4 251 073 B1**
(45) Date of publication and mention of the grant of the patent: **18.03.2026**
(21) Application number: 21898838.4
(22) Date of filing: 10.11.2021
(51) Int. Cl.: A61B 17/285

(54) **DEVICE FOR ENDOSCOPIC TISSUE CUTTING AND EXTRACTION FROM THE ABDOMEN**
VORRICHTUNG ZUM ENDOSKOPISCHEN SCHNEIDEN UND ENTFERNEN VON GEWEBE AUS DEM ABDOMEN
DISPOSITIF POUR DÉCOUPER ET EXTRAIRE PAR ENDOSCOPIE DES TISSUS DE L'ABDOMEN

(30) Priority: 30.11.2020 TR 202019309; 16.03.2021 TR 202104862
(43) Date of publication of application: 04.10.2023
(73) Proprietor: Ege Universitesi, 35100 Izmir (TR)
(72) Inventor: ARI, Sabahattin Anil, Bornova / Izmir (TR); AKDEMIR, Ali, Bornova / Izmir (TR); GENÇER, Gökçe Mehmet, Bornova / Izmir (TR)
(74) Representative: Potter Clarkson
(86) International application number: PCT/TR2021/051181
(87) International publication number: WO 2022/115078

(56) References cited:
- WO-A1-2017/222262
- CN-U- 202 821 529
- CN-U- 204 765 741
- CN-U- 210 521 059
- SU-A1- 904 684
- US-A1- 2016 059 427

## Description

### Technical Field of Invention

The invention relates to a device for endoscopic tissue cutting and extracting from the abdomen through the incisions, which enables the tissues/masses to be extracted from the abdomen in laparoscopic surgery and are larger in size than these incisions in which the surgery is performed.

### Prior Art

CN204765741 discloses a tissue sampling scissors comprising two blade portions which are polyhedrons with a triangular front face and a polygonal blade surface with a blade, and the front face of the two blade portions is a triangle after the two blade portions are engaged. SU904684A1 describes a type of forceps designed for medical use, specifically for removing membranes from the larynx.

CN202821529U discloses a pair of surgical scissors with half-spoon shaped blades.

Laparoscopic surgeries are performed through incisions ranging in width from 0.5 cm to 1 cm. After the surgery, much larger masses should be removed from the abdomen compared to these incisions. Removing the masses from the abdomen is a difficult and time-consuming process, often takes longer than the surgery itself and compels surgeons to perform open surgery. Therefore, patients are deprived of the comfort of laparoscopic surgery and
experience disadvantages such as risk of infection, pain, being away from work and social life for a long time. The invention enables the tissues/masses that are larger in size than the incisions in which the surgery is pe1formed and are wished to be extracted from the abdomen in laparoscopic surgery to be separated and extracted from these incisions.

Currently, extraction of masses from the abdomen in laparoscopic surgery is performed by;
- Separating into pieces with a scalpel,
- Using electrical devices called morcellators that are used to cut the tissue/mass with a rotational motion and extract it from the abdomen,
- Extracting the mass directly to the outside of the abdomen through a large incision, which is made as large as the size of the mass.

In the state of the art, trying to break up large tissues/masses with a scalpel through natural orifices or incisions that limit the surgeon's range of motion with a width of 1.5-2 cm is quite laborious, tiring, time-consuming and risky for patient health. This process can extend the duration of the surgery between 60 and 90 minutes. In addition, the risk of injury or damage to the surrounding tissues is high due to the aforementioned procedures.

In the use of morcellator disintegrating devices, there is a risk of spreading/scattering the tissue/mass that is tried to be removed into the abdomen in small pieces due to the rotational movement in the working principle. In operations where the morcellator device is used, if the removed tissue is malignant (cancer), the patient is faced with the risk of metastasis spreading to the entire abdomen. Due to the regretful experience, the use of this device has been banned in the United States of America, and it is required to be used in sterile bags in the European Union countries. However, even the use of these bags cannot completely prevent the spread of tissue into the abdomen.

Another way to extract the mass from the abdomen at the end of the operation is a large incision to be made in the abdomen. This incision eliminates all the advantages of laparoscopic surgery, increases the patient's pain and prolongs the hospital stay, increases the cost of the operation, creates a risk of infection, and therefore delays the patient's return to work/social life.

### Brief Description and Aims of the Invention

The invention provides a device according to claim 1. Further embodiments of the invention are provided in the dependent claims.

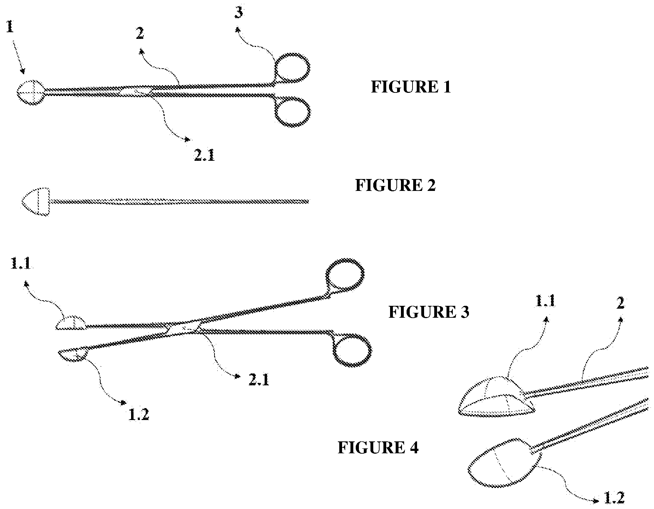

With the invention, the mass to be extracted from the abdomen after the laparoscopic operation can be easily disintegrated without an additional abdominal incision in hysterectomy (removal of the uterus) surgery, and through a 3 cm incision to be made on the top of the vagina in myomectomy or adnexal mass surgeries. The invention consists of two chambered parts (Figure 4), which are triangular when viewed from the top (Figure 2) with their cutter faces contacting each other when closed, and holder handles connected to these cutting chambers. The two cutting blades/chambers of the invention are designed to be able to enter into the abdomen through a 3 cm incision while the invention is in the closed position. When these blades enter into the abdomen, they are opened with the help of the retaining handles, the tissue/mass as large as the volume of the chamber is cut and this mass can be directly extracted from the abdomen by keeping the invention in the closed position. The tissue/mass can be extracted to the outside of the abdomen quickly and safely with repetitive movements. Since large-sized tissues/masses can be removed from the abdomen through a maximum 3 cm incision with the advantage of the invention, patients are protected from the possible risks of a large incision. Since the invention is designed to cut the tissue/mass directly (it does not rotate like the morcellator device) and to keep the cut piece in its own chamber, there is no risk of the tissue/mass spreading into the abdomen.

It is planned to produce the invention from stainless steel material that can be used in surgical operations, can be disinfected and is compatible with the human body.

The use of the invention in closed hysterectomy and myomectomy operations is envisioned. According to OECD data, the number of hysterectomies in 2017 was 106.012 for Germany, 47.771 for the United Kingdom, and 116.163 for Mexico. While the number of laparoscopic hysterectomies performed in Germany in 2005 was 6570, this number increased to 40.675 in 2017. For Turkey, the last data date is 2012 and the number of hysterectomies is 10.915 (https://stats.oecd.org/index.aspx?queryid=30167). The most common operation in the United States of America is hysterectomy. Today, the fact that patients desire to return to their work life as soon as possible and force surgeons to minimally invasive surgery. However, in this regard, extracting the tissues from the abdomen creates a problem.

After the prohibition of power morcellation by the FDA in 2014, the frequency of complications and the number of blood transfusions increased related to open surgeries performed in the United States. Thereupon, ACOG recommended open surgery or vaginal morcellation in sterile bags for large uteruses and myomas. In this recommendation, it is noted that attention should be paid to adjacent organ injuries (ACOG COMMITTEE OPINION Number 770). At this point where the technique is problematic, the invention is a strong candidate to be a frequently preferred surgical instrument with its new, unique and practical use properties. If the number of myomectomies is added to the number of hysterectomies, it is obvious that it will be of demand in large numbers annually in our country, the European Union and the United States. Again, according to OECD data, the number of hysterectomies and myomectomies to be performed in Turkey in 2022 is expected to be approximately 15.000. It is estimated that 30% of this will be done by closed methods. According to statistical data, in the case it becomes ready in 2022, the invention can be used in 4500 gynecological operations annually.

Laparoscopic surgeries are not a field of only gynecologists. Today, all surgeons want to perform operations with minimally invasive methods. Gallbladder, colon and esophagus operations in general surgery; prostate and kidney removal surgeries in urology, arthroscopic procedures in orthopedics are increasingly being performed laparoscopically. This increase brings with its difficulties in extracting the tissues. The invention can be used to safely extract large masses from small incisions after all abdominal surgeries.

### Definitions of Drawing Explaining the Invention

The figures and related explanations used in order to better explain the device for endoscopic tissue cutting and extraction from the abdomen developed with this invention are below.
Figure **1****.** Frontal view of the invention in closed position
Figure 2. Top view of the invention in closed position
Figure 3. Front view of the invention in open position
Figure **4****.** View of the chamber part and cutting surfaces of the invention in the open position

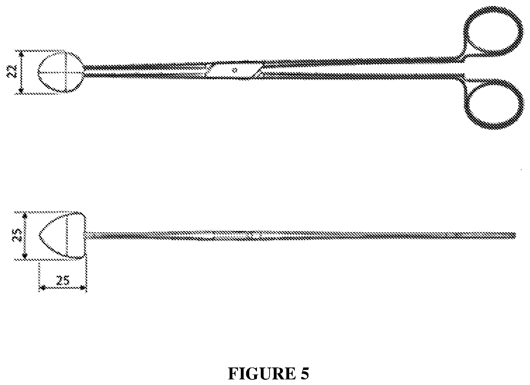

Figure 5. Basic design sizes of the invention (sizes are given in mm)

### Definition of Elements Constituting the Invention

In order to better explain the device for endoscopic tissue cutting and extraction from the abdomen developed with this invention, the parts and sections in the figures are numbered, and the corresponding definitions are given below.
1- Blade
   1.1- Top cutter with chamber
   1.2- Bottom cutter with chamber
2- Handle
   **2.1-** Connection Hole
3- Holder

### Detailed Description of the Invention

The invention provides a solution to the problem of extracting large masses from the abdomen through small incisions after laparoscopic surgery. Extraction of a large mass from the abdomen after laparoscopic surgery is possible by making additional incision in the size of the mass, using a morcellator device, dividing the mass into pieces with a scalpel through the top of the vagina in hysterectomy surgeries and through an additional incision on the top of the vagina in myomectomy and adnexal mass surgeries. A large incision has no place in the targeted laparoscopic approaches in minimally invasive surgery. The use of morcellator has been abandoned by many surgeons as it causes the tissues to be extracted to spread into the abdomen. The extraction of large masses from the abdomen is mostly done by dividing the mass through existing incisions with a scalpel. This procedure is both difficult and time-consuming and poses a risk of injury to the surrounding tissues. Using the invention instead of the existing devices proposes a safe, fast and easy solution. The invention consists of blade (1), handle (2) and holder (3). The blade part consists of two chamber cutters; a top cutter with a chamber (1.1) and a bottom cutter with a chamber (1.2), whose bases (sharp surfaces) face each other and triangularly shaped when viewed from above. In addition to its ease of use, thanks to this design, the invention will be able to enter the abdomen through an incision of about 3 cm on the top of the vagina, grasp and cut the mass to be extracted from the abdomen, and keep the cut piece in its own chamber (1.1, 1.2). Thus, the mass wished to be extracted can be removed without contacting other abdominal regions and the incision line. In addition, by means of the round design outside the blade (1), it does not harm the surrounding tissues. With the serial repetition of the process of cutting and extracting from the abdomen, large masses can be extracted from the abdomen quickly, safely and without contacting other parts of the abdomen and the incision site.

The invention consists of two identical parts. Each part has a blade (1), a handle (2) and a holder (3). The blade (1) of the invention is triangular when viewed from above and has a cutting surface at the base. The width, height and depth of the top cutter with chamber (1.1) and the bottom cutter chamber (1.2), are 25, 11 and 25 mm, respectively. The parts of the top cutter with chamber (1.1) and the bottom cutter with chamber (1.2) that face each other (contacting surfaces when the device is in closed position) are sharp and are designed to cut myoma or uterus. The circumference of the completely closed part formed by the closure of the blade (1) is designed with rounded curves so as not to damage the surrounding tissues. The blade (1) parts of the invention are connected with a handle (2). There is a connection hole (2.1) in the middle of the handles (2). After the connection holes (2.1) are overlapped, these handles (2) are mounted to each other with a screw or similar element. On the other end of the handle (2) part of the invention, there is the round holder (3) part into which the 1^{st} and 4^{th} fingers of the hand can slip. The invention is made of stainless-steel material that can be used in surgical operations and is compatible with the human body.

## Claims

1. A device for endoscopic tissue cutting and extraction from the abdomen, the device comprising
• a blade (1) which consists of two chamber cutters, top cutter with chamber (1.1) and bottom cutter with chamber (1.2), whose sharp surfaces face each other and are triangularly shaped when viewed from above,
• two handles (2) to which the top cutter with chamber (1.1) and bottom cutter with chamber (1.2) are connected, and
• two round holders (3) into which the 1^{st} and 4^{th} fingers of the hand can slip and to which the other end of the handles (2) is connected,
wherein the chamber cutters comprise a completely closed structure as a result of the round holders (3) being closed towards each other and the circumference of the completely closed structure formed by the closure of the blade (1) has rounded curves so as not to damage the surrounding tissues.

2. A device according to Claim 1, wherein the width, height and depth of said top cutter with chamber (1.1) and bottom cutter with chamber (1.2), are 25, 11 and 25 mm, respectively.

3. A device according to Claim 1, wherein the device comprises connection holes (2.1) realizing the connection of said handles (2) to each other.

4. A device according to Claim 3, wherein the mounting of said handles (2) to each other is made by means of screws passing through connection holes (2.1).

5. A device according to Claim 1, wherein the device is manufactured from stainless-steel material.

## Patentansprüche

1. Vorrichtung zum endoskopischen Schneiden und Entnehmen von Gewebe aus dem Bauchraum, die Vorrichtung umfassend
• eine Klinge (1), die aus zwei Kammermessern besteht, einem oberen Messer mit Kammer (1.1) und einem unteren Messer mit Kammer (1.2), deren scharfe Oberflächen einander zugewandt sind und von oben betrachtet dreieckig geformt sind,
• zwei Griffe (2), mit denen das obere Messer mit Kammer (1.1) und das untere Messer mit Kammer (1.2) verbunden sind, und
• zwei runde Halterungen (3), in die der erste und vierte Finger der Hand eingeführt werden können und mit denen das andere Ende der Griffe (2) verbunden ist, wobei die Kammermesser eine vollständig geschlossene Struktur aufweisen, da die runden Halterungen (3) zueinander geschlossen sind und der Umfang der vollständig geschlossenen Struktur, die durch das Schließen der Klinge (1) gebildet wird, abgerundete Kurven aufweist, um das umgebende Gewebe nicht zu beschädigen.

2. Vorrichtung nach Anspruch 1, wobei die Breite, Höhe und Tiefe des oberen Messers mit Kammer (1.1) und des unteren Messers mit Kammer (1.2) jeweils 25, 11 und 25 mm betragen.

3. Vorrichtung nach Anspruch 1, wobei die Vorrichtung Verbindungslöcher (2.1) umfasst, die die Verbindung der Griffe (2) miteinander ermöglichen.

4. Vorrichtung nach Anspruch 3, wobei die Befestigung der Griffe (2) aneinander mittels Schrauben erfolgt, die durch Verbindungslöcher (2.1) hindurchgeführt werden.

5. Vorrichtung nach Anspruch 1, wobei die Vorrichtung aus rostfreiem Stahl hergestellt ist.

## Revendications

1. Dispositif de découpe et d'extraction endoscopique de tissus abdominaux, le dispositif comprenant
• une lame (1) qui consiste en deux éléments de coupe à chambre, un élément de coupe supérieur à chambre (1.1) et un élément de coupe inférieur à chambre (1.2), dont les surfaces tranchantes se font face et sont de forme triangulaire en vue de dessus,
• deux poignées (2) auxquelles sont reliés l'élément de coupe supérieur à chambre (1.1) et l'élément de coupe inférieur à chambre (1.2), et
• deux porte-doigts circulaires (3) dans lesquels le 1^{er} et le 4^{e} doigt de la main peuvent s'insérer et auxquels est reliée l'autre extrémité des poignées (2),
dans lequel les éléments de coupe à chambre comprennent une structure complètement fermée du fait que les porte-doigts circulaires (3) sont rapprochés l'un de l'autre, et le périmètre de la structure complètement fermée formée par la fermeture de la lame (1) présente des courbures arrondies de manière à ne pas endommager les tissus environnants.

2. Dispositif selon la revendication 1, dans lequel la largeur, la hauteur et la profondeur dudit élément de coupe supérieur à chambre (1.1) et de l'élément de coupe inférieur à chambre (1.2) sont respectivement de 25, 11 et 25 mm.

3. Dispositif selon la revendication 1, dans lequel le dispositif comprend des orifices de liaison (2.1) assurant la liaison desdites poignées (2) entre elles.

4. Dispositif selon la revendication 3, dans lequel le montage desdites poignées (2) l'une à l'autre est réalisé au moyen de vis passant à travers des orifices de liaison (2.1).

5. Dispositif selon la revendication 1, dans lequel le dispositif est fabriqué en acier inoxydable.
